# EUROPÄISCHE PATENTSCHRIFT

(11) **EP 3 713 915 B1**
(45) Veröffentlichungstag und Bekanntmachung des Hinweises auf die Patenterteilung: **19.10.2022**
(21) Anmeldenummer: 18803625.5
(22) Anmeldetag: 13.11.2018
(51) Int. Cl.: C07D 207/08

(54) **VERFAHREN ZUR HERSTELLUNG VON BISPYRROLIDIN-VERBINDUNGEN**
METHOD FOR PRODUCING BISPYRROLIDINE COMPOUNDS
PROCÉDÉ DE PRÉPARATION DE COMPOSÉS DE BISPYRROLIDINE

(30) Priorität: 23.11.2017 EP 17203292
(43) Veröffentlichungstag der Anmeldung: 30.09.2020
(73) Patentinhaber: BASF SE, 67056 Ludwigshafen am Rhein (DE)
(72) Erfinder: KLOPSCH, Rainer, 67056 Ludwigshafen (DE); ZIPFEL, Hannes Ferdinand, 67056 Ludwigshafen (DE); GORDILLO BOLONIO, Alvaro, 67056 Ludwigshafen (DE)
(74) Vertreter: BASF IP Association
(86) Internationale Anmeldenummer: PCT/EP2018/080993
(87) Internationale Veröffentlichungsnummer: WO 2019/101568

(56) Entgegenhaltungen:
- WO-A1-2012/068001
- CN-B- 101 200 445
- DE-A1-102014 215 388

## Beschreibung

Gegenstand der vorliegenden Anmeldung ist ein Verfahren zur Herstellung von Bispyrrolidin-Verbindungen der Formel I, bei denen in einem ersten Schritt γ-Butyrolacton mit Diaminen der Formel II zu Bisamiden der Formel III umgesetzt werden, wobei die Amidfunktion der Bisamide der Formel III anschließend im Schritt II) teilweise oder vollständig zu Bispyrrolidin-Verbindungen der Formel I katalytisch hydriert werden.

Es besteht ein großer Bedarf an Verbindungen die als Katalysator bei der Herstellung von Polyurethanschäumen eingesetzt werden und anschließend keine oder geringe Emissionen verursachen. Dabei geht es um Emissionen des Katalysators selber, sowie Emissionen von Abbauprodukten wie z.B. Amine, DMF und Formaldehyd.

Katalysatoren, die sich bei der Reaktion in das Polyurethannetzwerk einbauen und sich damit späterer Emission entziehen, sind zahlreich in der Industrie verfügbar. Katalysatoren, die geringe Emmissionen in Form von Amin-, DMF- und Formaldehydemissionen freisetzen, die für Amine zwischen ≥0µg/m3 und ≤40µg/m3, für DMF im Bereich von ≥0 bis ≤5ppm und für Formaldehyd innerhalb oder unterhalb der von den Schaumherstellern und der Möbelindustrie in Europa und der USA im Rahmen des freiwillig auferlegten Programms "CertiPUR" festgelegten Grenzen für Aldehydemmisionen, insbesondere Formaldehydemissionen liegen und in WO 2016/020139 von Seite 4 Zeile 12 bis Seite 6, Zeile 11 näher beschrieben, sind jedoch nicht breit verfügbar, insbesondere, wenn sie gleichzeitig hinsichtlich der katalytischen Aktivität dem Stand der Technik in nichts nachstehen sollen oder sogar besser sein sollen.

In WO 2016/020139 werden Bispyrrolidinverbindungen der Formel I hergestellt die als Katalysatoren bei der Polyurethanherstellung für Weich- und Hartschäume eingesetzt werden. Hierbei werden die Bispyrrolidinverbindungen jedoch ausgehend von Pyrrolidin und 2-Chlorethylether oder Pyrrolidin und Diethylenglykol in Gegenwart eines Ru-Katalysators umgesetzt. Die Umsetzung ausgehend von γ-Butyrolacton und den Diaminen nach Formel II wird hierbei nicht offenbart.

In WO 2015/20048 werden Polyurethanzusammensetzungen beschrieben, die als Katalysator auch symmetrische Bispyrrolidin-ether-Verbindungen einsetzen. Die Herstellung der expliziten Bispyrrolidin-ether-Verbindungen der Formel I werden jedoch hier nicht offenbart.

In US 9351484 B2 wird die Herstellung von symmetrischen und unsymmetrischen Bis(1-Alkylpyrrolidin-2-on)ethern beschrieben. Dabei werden die entsprechenden N-Hydroxyalkylpyrrolidone in Gegenwart von sauren Katalysatoren dehydriert und das entstehende Wasser entfernt. Bei dem erfindungsgemäßen Verfahren dagegen werden keine Katalysatoren hinzugegeben, die anschließend in einem separaten Schritt wieder entfernt werden müssen. Des Weiteren ist das erfindungsgemäße Verfahren wenigstens um eine Stufe kürzer und damit auch ökonomischer als das in US 9351484 B2 beschriebene.

M- F. Shostakovska et al. in Seriya Khimicheskaya (1961),910-13 beschreibt die Bildung von symmetrischen und unsymmetrischen Bis(1-Alkylpyrrolidin-2-on)ether-Verbindungen ausgehend von den N-Hydroxyalkylpyrrolidonen in Gegenwart von Carbonsäuren und Schwefelsäure und anschließender azeotropischen Destillation.

EP 0 693 466 B1 beschreibt unter anderem die Herstellung von 1,1'-(Oxybis(ethan-2,1-diyl)) bis (pyrrolidin-2-on) welches einem Bisamid der Formel III entspricht. Allerdings wird dieses Bisamid durch eine Williamson-Ethersynthese aus 2-Pyrrolidinon, Kaliumhydoxid, einem Katalysator und Bis-(2-Chlorethyl)-ether hergestellt. Eine Umsetzung aus γ-Butyrolcaton und Verbindungen der Formel II zum entsprechenden Bisamid wird nicht offenbart. Das in EP 0693 466 B1 beschriebene Bisamid wird als Lösungsmittel zur Halogenierung von aromatischen Verbindungen eingesetzt.

EP 0 930 293 B1 beschreibt die Herstellung von N-Vinylpyrrolidon (NVP) durch Dehydratisierung von N-(2-Hydroxyethyl)pyrrolidon (HEP) unter Einsatz eines Katalysators wobei auch Bisamide der Formel III als Nebenprodukte entstehen. Die Herstellung von Bisamiden der Formel III durch Umsatz von γ-Butyrolcaton und Verbindungen der Formel II wird nicht beschrieben.

Reppe beschreibt in Liebigs Annalen der Chemie 1955, Seite 102-104 die Umsetzung von Tetrahydrofuran und 1,4-Butandiol mit Ammoniak und primären Aminen zu verschiedenen Produkten unter anderem auch die Herstellung von Dipyrrolidylalkanen. Der Einsatz von γ-Butyrolacton und Einsatz eines Katalysators zur Hydrierung im zweiten Schritt wird jedoch nicht beschrieben.

In DE 4008120, CN 101948448, US 2525884 und vielen weitere Schriften wird die Bildung von Bispyrrolidinen grundsätzlich beschrieben. Hierbei werden jedoch nur gute Selektivitäten mit hohen Aminüberschüssen erreicht.

J. Falkstorp offenbart in Acta Chemica Scandinavica (1957), 11, 1698-1705 die Herstellung von 1,1'-(oxybis(ethan-2,1-diyi))dipyrroiidin über Williamson Ethersynthese ausgehend von 2-(pyrrolidin-1-yl)ethan-1-ol in Gegenwart von 1-(2-chloroethyl)pyrrolidin und einem Katalysator. Ein Hydrierverfahren wie in Schritt II des erfindungsgemäßen Verfahrens wird nicht beschrieben.

Ken-ichi Shimizu et al beschreiben in ChemistrySelect 2016, 4, 736-740 die Hydrierung von tertiären Amiden mit Pt-Katalysatoren, die auf lewissauren Trägern aufgebracht sind. Hierbei wird auch die Hydrierung von Pyrrolidin-2-on-Derivaten beschrieben. Einsatz von Bisamiden der Formel III in einer solchen Hydrierung wird jedoch nicht offenbart.

Kiyotomi Kaneda et al. beschreiben in Angewandte Chemie Int. Ed. 2017, 56, 9381-9385 die Hydrierung von Amiden zu den entsprechenden Aminen unter milden Bedingungen in Gegenwart von Pt/V Katalysatoren, die auf Hydroxyapatite (HAP) als Träger aufgetragen sind, wie z.B. auch die Hydrierung von N-Acetylpyrrolidin zum entsprechenden N-Ethylpyrrolidin. Die Hydrierung des Bisamides der Formel III wird jedoch nicht offenbart.

Keines der im Stand der Technik beschriebenen Verfahren offenbart die Möglichkeit Bispyrrolidin-Verbindungen der Formel I in nur zwei Schritten ausgehend von γ-Butyrolacton mit Diaminen der Formel II zu Bisamiden der Formel III und anschließender katalytischer Hydrierung zu den Bisyprrolidin-Verbindungen der Formel I ohne Aminüberschuss herzustellen.

Die Aufgabe der vorliegenden Erfindung ist es daher ein einfaches Verfahren zur Herstellung von Bispyrrolidin-Verbindungen der Formel I ausgehend von günstigen einfachen Edukten wie γ-Butyrolacton in möglichst wenig Schritten und ohne Aminüberschüsse herzustellen.

Diese Aufgabe wird gelöst durch ein Verfahren zur Herstellung von Bispyrrolidin-Verbindungen der Formel I 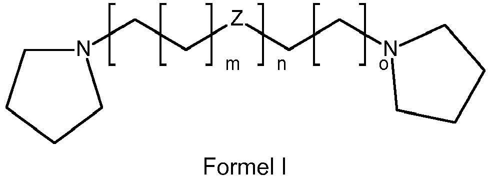 mit
- Z: ausgewählt aus der Gruppe von Sauerstoff und Kohlenstoff,

- n: eine ganze natürliche Zahl im Bereich von 0 bis 9 ist, m und o unabhängig voneinander eine natürliche Zahl im Bereich von 1 bis 12 sind, wobei in Schritt I) γ-Butyrolacton mit Diaminen der Formel II 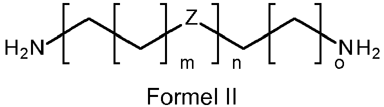
wobei Z, n, m und o die obige Bedeutung haben, zu Bisamiden der Formel III 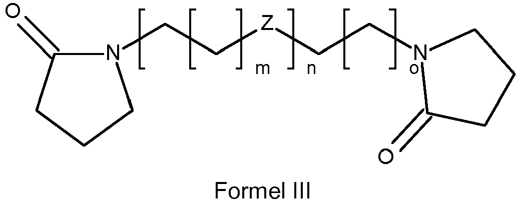 Umgesetzt werden und anschließend im Schritt II) die Bisamide der Formel III katalytisch zu den Bispyrrolidin-Verbindungen der Formel I hydriert werden.

Vorteilhaft ist das erfindungsgemäße Verfahren, wenn die Umsetzung des γ-Butyrolactons mit der Verbindung II in Schritt I) bei Temperaturen im Bereich von 100 bis 300 °C durchgeführt wird und gleichzeitig oder anschließend das überschüssige Wasser destillativ entfernt wird.

Vorteilhaft ist das erfindungsgemäße Verfahren, wenn der Schritt II) in Gegenwart eines Hydrierkatalysators durchgeführt wird, bei dem der Hydrierkatalysator wenigstens ein Metall ausgewählt aus der Gruppe von Cu, Cr, Ni, Co, Fe, Pt, Pd, Re, Ru, und Rh enthält.

Vorteilhaft ist das erfindungsgemäße Verfahren, wenn in Schritt II) mit einem heterogenen Katalysator gearbeitet wird.

Vorteilhaft ist das erfindungsgemäße Verfahren, wenn Schritt II) in Gegenwart eins Raney Cobalt, Raney Nickel oder dotierten oder undotierten Katalysatortyps von Cu-Cr durchgeführt wird.

Vorteilhaft ist das erfindungsgemäße Verfahren, wenn die Hydrierung in Schritt II) in Gegenwart eines Raney Cobalt Katalysators oder eines dotierten Katalysatortyps von Cu-Cr bei Temperaturen im Bereich von 100 bis 300°C und bei einem Wasserstoffdruck im Bereich von 100 bis 350 bar durchgeführt wird.

Vorteilhaft ist das erfindungsgemäße Verfahren, wenn Z Sauerstoff ist und n 1 oder 2 ist und m=o=1 ist.

Vorteilhaft ist das erfindungsgemäße Verfahren, wenn die Verbindungen der Formel IB bei denen unabhängig voneinander -X1 und -X2 ausgewählt sind aus der Gruppe von H und Doppelbindung Sauerstoff, wobei aber wenigstens -X1 oder -X2 für Wasserstoff steht und die nach Schritt II neben den Bispyrrolidin-Verbindungen der Formel I erhalten werden können in einem weiteren Schritt III) noch einmal katalytisch hydriert werden.

Vorteilhaft ist das erfindungsgemäße Verfahren, wenn die Hydrierung von Schritt II) und III) mit dem gleichen Katalysator durchgeführt wird.

Vorteilhaft ist das erfindungsgemäße Verfahren, wenn die Hydrierung von Schritt II) und III) in einem Schritt erfolgen kann.

Vorteilhaft ist das erfindungsgemäße Verfahren, wenn die Verbindung nach Formel I der Bispyrrolidin-ether der Formel IA ist

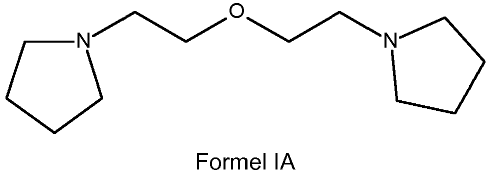

Mit dem erfindungsgemäßen Verfahren werden Bispyrrolidin-Verbindungen der Formel I 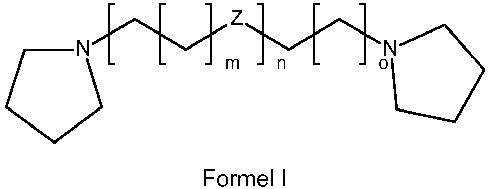 mit Z ausgewählt aus der Gruppe von O und C. Bevorzugt ist = O.

Das n ist eine ganze natürliche Zahl im Bereich von 0 bis 9, bevorzugt im Bereich von 1 bis 5, besonders bevorzugt im Bereich von 1 bis 3, ganz besonders bevorzugt im Bereich von 1 oder 2. Die Indices m und o können unabhängig voneinander eine ganze natürliche Zahl im Bereich von 1 bis 12 sein. In Bispyrrolidin-Verbindungen der Formel I ist m von n abhängig. Bevorzugte Verbindungen für den Fall, dass n=1 ist, sind solche bei denen m und o unabhängig voneinander eine ganze Zahl im Bereich von 1 bis 12 sind. Besonders bevorzugt sind in diesem Fall für n=1 m und o die gleiche Zahl im Bereich von 1 bis 12. Bevorzugte Verbindungen für den Fall, dass n=2 bis 9 ist, sind solche bei denen m=1 ist und o kann eine Zahl im Bereich von 1 bis 12 sein. Besonders bevorzugt sind in diesem Fall für n=2-9 und m und o=1. Bevorzugt sind Bispyrrolin-Verbindungen der Formel I ausgewählt aus der Gruppe bei denen n, m und o= 1 sind, n=2 mit m=1 und o=1, n=3 mit m=1 und o=1 und Verbindungen bei denen n=1 und m und o identisch sind. Besonders bevorzugt sind Verbindungen der Formel I bei denen n, m und o= 1 sind. Bevorzugte Verbindungen der Formel I sind ausgewählt aus der Gruppe von

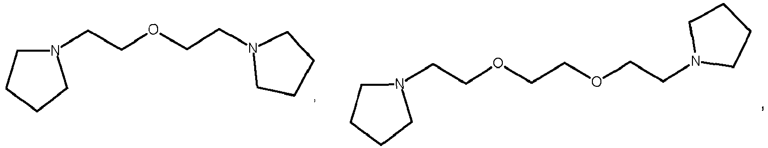

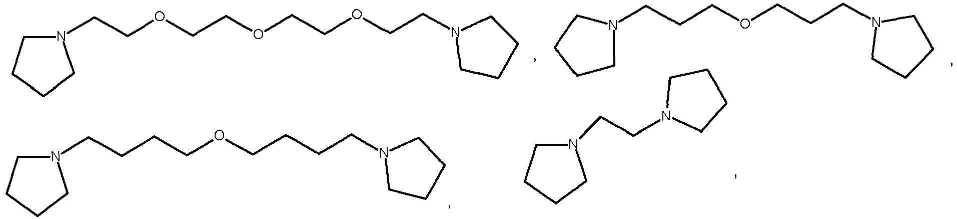

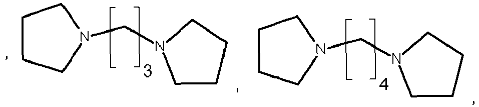

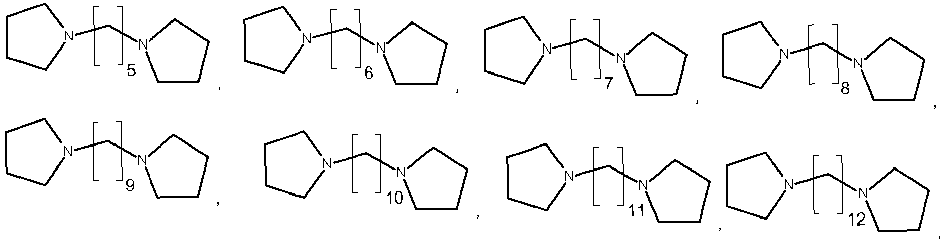

Bevorzugte Bispyrrolidin-Verbindungen der Formel I sind solche bei denen Z= O oder NR und n und m=1 und o eine ganze Zahl im Bereich von 1 bis 12 sind. Besonders bevorzugt ist die Bispyrrolidin-Verbindung der Formel IA

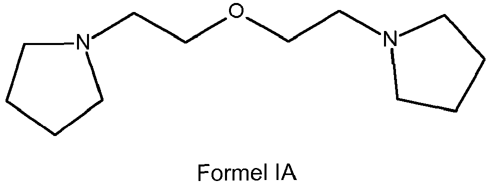

In dem erfindungsgemäßen Verfahren wird in Schritt I γ-Butyrolacton mit dem Diamin der Formel II umgesetzt. Bei dem Diamin der Formel II haben Z, m und o die gleiche Bedeutung wie bei den Bispyrrolidin-Verbindungen der Formel I.

Die Umsetzung des Diamins der Formel II mit dem γ-Butyrolacton kann in Gegenwart eines Lösungsmittels erfolgen. Bevorzugt wird die Umsetzung jedoch ohne Lösungsmittel durchgeführt.

Die Umsetzung von γ-Butyrolacton mit dem Diamin der Formel II erfolgt bei Temperaturen im Bereich von 150 bis 400°C, bevorzugt im Bereich von 200-350, besonders bevorzugt im Bereich von 250-300 °C. Das dabei entstehende Reaktionswasser kann dabei gleichzeitig destillativ entfernt werden.

Im Schritt II des erfindungsgemäßen Verfahrens wird das aus Schritt I erhaltene Bisamid der Formel III katalytisch hydriert. Der Hydrierung kann ein Trocknungsschritt wie Destillation oder eine herkömmliche Trocknung mit Trocknungsmittel vorgelagert sein, muss aber nicht. Bei den Bisamiden der Formel III haben Z, m und o die gleiche Bedeutung wie bei den Bispyrrolidin-Verbindungen der Formel I.

Als Katalysator im Schritt II kann jeder Katalysator verwendet werden, der befähigt ist Amidgruppen zu den entsprechenden sekundären Aminen zu hydrieren. Die Hydrierung im Schritt II kann sowohl homogenen als auch heterogen katalysiert sein. Bevorzugt ist der Einsatz von heterogenen Katalysatoren. Die Katalysatoren für die Hydrierung enthalten wenigstens ein Metall ausgewählt aus der Gruppe von Cu, Cr, Ni, Co, Fe, Pt, Pd, Re, Ru und Rh. Die Katalysatoren können auch noch mit weiteren Metallen ausgewählt aus der Gruppe von Alkali-, Erdalkalimetallen und Metallen aus der 3. bis 8. Nebengruppe des Periodensystems dotiert sein. Bevorzugt sind die Dotierungsmetalle aus der Gruppe von Li, Na, K, Mg, Ca, Ba, W, Mo, V, Mn, P, B und Sn ausgewählt. Die Katalysatoren können dabei auf unterschiedlichen Trägermaterialien aufgebracht sein. Die Trägermaterialien sind dabei ausgewählt aus der Gruppe von Siliziumdioxid, Kohle, Titandioxid, Aluminiumoxid Al₂O₃, Nioboxid Nb₂O₅, Ceroxid CeO₂ und Siliciumcarbid SIC und Zirkonoxid ZrO₂. Auch diese Träger können wie auch die Metalle selber mit den gleichen Dotiermetallen wie oben beschrieben dotiert sein. Neben den einzelnen dotierten oder undotierten aktiven Metallen auf den dotierten oder undotierten Trägermaterialien sind auch bimetallische Katalysatoren einsetztbar. Diese sind dabei ausgewählt aus der Gruppe von Pt-Nb, Pt-V, Rh-Mo, Ru-Mo, Ru-Re, Rh-Re, Pt-Re, Pd-Re, Rh-W und Cu-Cr (z.B. Kupferchromit Katalysatoren). Auch diese bimetallischen Katalysatoren können sowohl beim aktiven Metall als auch im Träger mit den obigen Dotierstoffen dotiert sein. Besonders bevorzugte Katalysatoren sind ausgewählt aus der Gruppe von Raney Co, Raney Ni, Katalysatoren von dotiertem oder undotiertem Kupferchromittyp wie Cu-Cr, Cu-Cr/Mn und Cu-Cr/Ba. Ganz besonders bevorzugte Katalysatoren sind Raney Co und der Katalysator vom dotierten Kupferchromittyp Cu-Cr/Ba.

Während der Hydrierung in Schritt II wird in Gegenwart von reinem und/oder synthetischem Wasserstoff oder Gemischen aus Wasserstoff mit anderen Inertgasen, ausgewählt aus der Gruppe Stickstoff und Argon gearbeitet. Die Hydrierung kann in Gegenwart von Lösungsmitteln ausgewählt aus der Gruppe von Ammoniak, Ethern, Alkoholen, Kohlenwasserstoffen, Alkylaromaten und Mischungen dieser Verbindungen. Bevorzugte Ether sind ausgewählt aus der Gruppe von Glyme, Diglyme, Proglyme, THF und Dioxan. Bevorzugte Alkohole sind ausgewählt aus der Gruppe von Methanol, Ethanol, n-Propanol, i-Propanol, n-Butanol, sek.-Butanol, IsoButanol, tert.-butanol, 1-Pentanol, 2-Pentanol, 3-Pentanol, 1-Hexanol, 2-Hexanol, 3-Hexanol, Heptanol, Ethylhexanol und Propylheptanol. Bevorzugte Kohlenwasserstoffe sind ausgewählt aus der Gruppe von Butan, Pentan, Hexan, Heptan, Octan, Nonan, Decan, Undecan, Dodecan, lineare und verzweigte Isomere und Mischungen davon. Bevorzugte Alkylaromaten sind ausgewählt aus der Gruppe von Benzol, Toluol und Xylol.

Bevorzugte Mischungen der Lösungsmittel sind ausgewählt aus der Gruppe von Ether mit Alkoholen, Alkoholen und Kohlenwasserstoffen, Alkoholen und Alkylaromaten, Kohlenwasserstoffen und Alkylaromaten, wobei die Alkohole und Kohlenwasserstoffe aliphatisch, cyclisch oder cycloaliphatisch sein können. Während der Hydrierung in Schritt II können auch noch weitere Additive enthalten sein. Diese sind ausgewählt aus der Gruppe von alkylierten Aminen, wobei die Amine mono-, di- oder trialkyliert sein können. Beispiele für Monoamine sind Methylamin, Ethylamin, Propylamin, Butylamin, Pentylamin, Hexylamin, Heptylamin, Octylamin, Nonylamin, Decylamin, Undecylamin, Dodecylamin. Beispiele für Dialkylamine sind Dimethylamin, Diethylamin, Dipropylamin, Dibutylamin, Dipentylamin, Dihexylamin, Diheptylamin, Dioctylamin, Dinonylamin, Didecylamin, Diundecylamin, Didodecylamin. Beispiele für Trialkylamine sind Trimethylamin, Triethylamin, Tripropylamin, Tributylamin, Tripentylamin, Trihexylamin, Triheptylamin, Trioctylamin, Trinonylamin, Tridecylamin, Triundecylamin, Tridodecylamin. Weitere Substituenten für Amine können lineare oder verzweigte oder cyclische C₁-C₂₀-Alkylgruppe und Alkoholate sein. Die linearen oder verzweigten oder cyclischen C₁-C₂₀Alkylgruppen sind dabei die gleichen linearen oder verzweigten oder cyclischen C₁-C₂₀Gruppen, die auch für R in der Formel I eingesetzt werden können. Die Temperatur liegt bei der Hydrierung im Bereich von 50 bis 300 °C bevorzugt im Bereich von 100 bis 250 °C, besonders bevorzugt im Bereich von 150 bis 250°C. Der Druck während der Hydrierung liegt im Bereich von 100 bis 350 bar, bevorzugt im Bereich von 150 bis 300 bar, besonders bevorzugt im Bereich von 150 bis 250 bar.

Das aus Schritt II erhaltene Produkt kann dann eine Mischung aus den möglichen Bispyrrolidin-Verbindungen der Formel I und Verbindungen der Formel IB sein.

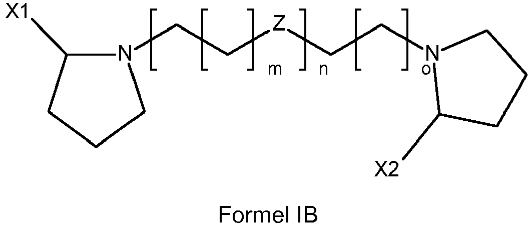

Bei denen unabhängig voneinander -X1 und -X2 ausgewählt sind aus der Gruppe von H und Doppelbindung Sauerstoff, wobei aber wenigstens -X1 oder -X2 Wasserstoff ist. Das Produkt aus dem erfindungsgemäßen Schritt II kann jedoch auch vollständig hydriert sein, so dass bereits nach Schritt II die Bispyrrolidin-Verbindungen der Formel I erhältlich sind. Für diesen Fall entfällt der erfindungsgemäße Schritt III.

Bei dem erfindungsgemäßen Schritt III handelt es sich um eine weitere Hydrierung der in Schritt II erhaltenen Verbindungen der Formel IB bei denen unabhängig voneinander -X1 und -X2 ausgewählt sind aus der Gruppe von H und Doppelbindung Sauerstoff, wobei aber wenigstens -X1 oder -X2 für Wasserstoff steht.

Auch während dieser Hydrierung wird in Gegenwart von reinem Wasserstoff oder Gemischen aus Wasserstoff und anderen Inertgasen ausgewählt aus der Gruppe von Stickstoff und Argon gearbeitet. Temperatur und Druck liegen sind dabei die gleichen bevorzugten Temperatur- und Druckbereiche wie bei Schritt II. Die in Schritt III eingestellten Temperaturen und Drücke müssen jedoch nicht mit denen in Schritt II identisch sein.

Als Hydrierungskatalysatoren sind hier die gleichen Katalysatoren einsetzbar wie für den Verfahrensschritt II. Bevorzugt ist für Verfahrensschritt III die Hydrierung mit einem heterogenen Katalysator. Besonders bevorzugt ist die Hydrierung in Gegenwart von Raney Co oder dem dotiertem Kupferchromittyp Cu-Cr/Ba. Wenn der Katalysator, Temperatur und Druck identisch in Schritt II und III sind, dann können diese beiden Schritte auch in Schritt II zusammengefasst werden.

Nach dem Schritt II und/oder Schritt III kann das erhaltene Gemisch, das die Verbindungen der Formel I enthält noch destillativ aufgearbeitet werden. Hierfür sind jegliche dem Fachmann bekannte Destillationskolonnen möglich. Die destillative Aufarbeitung erfolgt bevorzugt unter vermindertem Druck und bei erhöhten Temperaturen und einer oder mehrerer Kolonnen mit mehreren theoretischen Böden. Bevorzugt sind destillative Aufarbeitungen bei einem Druck im Bereich von 10 bis 150 mbar, Temperaturen im Bereich von 60 bis 230°C und Kolonnen mit einer theoretischen Bodenzahl im Bereich von 25- 30.

Beispiele
- Beispiel 1:: Verfahren zur Herstellung von 1,1'-(Oxybis(ethan-2,1-diyl))dipyrrolidin.
- Schritt I:: Herstellung von 1,1'-(Oxybis(ethan-2,1-diyl))bis(pyrrolidin-2-on)

Ein Kolben wird mit 100g 2,2'-Oxybis(ethan-1-amin) befüllt und auf 100 °C erhitzt. 165 g von γ-Butyrolacton (GBL) wird tropfenweise zugeführt und die Lösung anschließend 2 h gerührt. Nach vollständiger Zugabe wird die Mischung für weitere 2h bei 100°C gerührt. Anschließend wird die Mischung auf 220°C für 10 h erhitzt und dann auf 240°C für weitere 14h erhitzt, gleichzeitig wird Wasser destillativ entfernt. Nach Abkühlen wurde die Mischung gaschromatographisch untersucht. Die Mischung enthält 94,1%(F) von 1,1'-(Oxybis(ethane-2,1-diyl)) bis (pyrrolidin-2-on.
- Schritt II:: Herstellung von einem Gemisch aus 1,1'-(Oxybis(ethan-2,1-diyl))dipyrrolidin und 1-(2-(2-(Pyrrolidin-1-yl)ethoxy)ethyl)pyrrolidin-2-on.

10 g von Grace Raney Cobalt 2724 (50% slurry in H₂O) als Katalysator wird mit Tetrahydrofuran(THF) wasserfrei gewaschen und in einen 300mL Autoklaven überführt. Der Autoklav wird mit 50 g 1,1'-(Oxybis(ethan-2,1-diyl)) bis (pyrrolidin-2-on aus Schritt I befüllt und anschließend verschlossen. Der Autoklav wird mit 180 bar H₂ beaufschlagt und die Mischung wird für 48 h auf 170 °C erhitzt. Nach dem Abkühlen und Entspannen wird die erhaltene Mischung gaschromatographisch analysiert. Die Mischung enthält 36,7% (Fl) von 1,1'-(Oxybis(ethane-2,1-diyl))dipyrrolidine und 47,2% (Fl) von 1-(2-(2-(Pyrrolidin-1-yl)ethoxy)ethyl)pyrrolidin-2-one.

### Schritt III: Herstellung von 1,1'-(Oxybis(ethane-2,1-diyl))dipyrrolidin

5 g von Grace Raney Cobalt 2724 (50% slurry in H₂O) als Katalysator wird wasserfrei mit THF gewaschen und in einen 300ml Autoklaven überführt. Der Autoklave wird mit 50g von 1-(2-(2-(Pyrrolidin-1-yl)ethoxy)ethyl)pyrrolidin-2-on aus Schritt II befüllt und anschließend verschlossen. Der Autoklave wird mit 180 bar H₂ beaufschlagt und die Mischung wird auf 170 °C für 48 h erhitzt. Nach dem Abkühlen und Belüften wird die erhaltene Mischung gaschromatographisch analysiert. Die Mischung enthält 81,7% (Fl) von 1,1'-(Oxybis(ethane-2,1-diyl))dipyrrolidin.

### Allgemeine Vorschriften für die Ergebnisse der Tabellen 3-5 für die Hydrierung

Hydrierung des Bisamides (1,1'-(Oxybis(ethane-2,1-diyl)) bis (pyrrolidin-2-on) mit Raney-Co (Ra-Co):
Bisamid (100 g), Ra-Co (5 g) und ggf. die in den Tabellen 3-5 prozentuale Menge an Lösemittel und/oder Additiv, wobei der Prozentsatz den Anteil des Lösungsmittels und/oder Additives an der Gesamtsumme angibt, werden in einem Autoklaven (Innenvolumen 300mL) gefüllt. Der gefüllte Reaktor wird vorsichtig mit Wasserstoff bis zum in den Tabellen 3-5 angegebenen Druck gefüllt. Die Reaktion wird bei der in den Tabellen 3-5 angegebenen Temperatur mit 500 rpm gerührt. Nach Erreichen der in den Tabellen 3-5 angegebenen Reaktionstemperatur wird für eine in den Tabellen 3-5 angegebene Zeitspannen bei den Reaktionsbedingungen weitergerührt. Nach der in den Tabellen 3-5 angegebenen Zeitspanne wird der Reaktor auf Raumtemperatur abgekühlt, entspannt und geöffnet. Es wird eine dunkle Lösung mit dem festen Katalysator erhalten. Zum Zwecke der Analyse wird eine Probe gaschromatographisch untersucht.

Hydrierung des Bisamides (1,1'-(Oxybis(ethane-2,1-diyl)) bis (pyrrolidin-2-on) mit dem Katalysatoren der Tabelle 3 und 4, die nicht Raney-Co sind:
Bisamid (50 g), Katalysator (5 g) und die in den Tabellen 3-5 prozentuale Menge an Lösemittel, wobei sich der Prozentsatz den Anteil des Lösungsmittels an der Gesamtmischung angibt, werden in einem Autoklaven (Innenvolumen 300 mL) gefüllt. Der gefüllte Reaktor wird vorsichtig mit Wasserstoff bis zum in den Tabellen 3-5 angegebenen Druck gefüllt. Die Reaktion wird auf die in den Tabellen 3-5 angegebene Reaktionstemperatur erhitzt und mit 500rpm gerührt.

Nach Erreichen der angestrebten Reaktionstemperatur wird für einen in den Tabellen 3-5 angegebenen Zeitraum bei den Reaktionsbedingungen weitergerührt. Nach Ablauf dieser in den Tabellen 3-5 angegebenen Reaktionszeit wird der Reaktor auf Raumtemperatur abgekühlt, entspannt und geöffnet. Es wird eine dunkle Lösung mit dem festen Katalysator erhalten. Zum Zwecke der Analyse wird eine Probe gaschromatographisch untersucht.

Kontinuierliche Hydrierung des Bisamides (1,1'-(Oxybis(ethan-2,1-diyl)) bis (pyrrolidin-2-on) mit Ra-Co gemäß Tabelle 5:
Ein 70 mL Rohrreaktor wird mit Hydrierkatalysator gefüllt und der Katalysator wird gegebenenfalls im Wasserstoffstrom und unter Wärmezufuhr aktiviert. Nach der Aktivierung wird der Reaktor kontinuierlich unter der in der Tabelle 5 angegebenen Temperatur und beim in den Tabelle 5 angegebenen Druck mit einer Mischung aus Wasserstoff, (1,1'-(Oxybis(ethan-2,1-diyl)) bis (pyrrolidin-2-on) und gegebenenfalls Lösungsmittel gemäß Angaben in der Tabellen 5 beschickt und der Reaktionsaustrag wird zur Analyse gaschromatographisch untersucht.

Tabelle 1 zeigt die Zusammensetzung der eingesetzten Katalysatoren des Kupferchromittyps. Tabelle 2 umfasst Temperatur, Druck, Lösungsmittel und Additive in denen die bevorzugten Katalysatorsysteme für die Hydrierung des erfindungsgemäßen Verfahrens eingesetzt werden.

**Tabelle 1:**

| BASF Bezeichnung | Metall 1 | Gew.-% | Metall 2 | Gew.-% | Dotierstoff | Gew.-% |
|---|---|---|---|---|---|---|
| Cu 0202 P | CuO | 83 | Cr₂O₃ | 17 | - | - |
| Cu 0396 P (E-396) | CuO | 48 | Cr₂O₃ | 48 | MnO₂ | 4 |
| Cu 1800P | CuO | 52 | Cr₂O₃ | 48 | - | - |
| Cu 1885 P | CuO | 53 | Cr₂O₃ | 47 | - | - |
| Cu 1950 P | CuO | 47 | Cr₂O₃ | 46 | MnO₂ | 5,5 |
| E 108 P | CuO | 44 | Cr₂O₃ | 45 | BaO | 11 |

**Tablle 2:**

| Katalysatorsystem | Temperaturbereich (°C) | Druckbereich (bar) | Lösungsmittel /Additiv |
|---|---|---|---|
| Raney-Co | 170-240 | 150-200 | Dibutylamin |
| Cu 0202 P | 170-270 | 100-300 | Hexan |
| E-396 | 170-270 | 100-300 | Hexan |
| Cu 1950 P | 170-270 | 100-300 | Hexan |
| E 108 P | 170-270 | 100-300 | Hexan |
| Cu 1885 P | 170-270 | 100-300 | Hexan |

Tabelle 3-5 zeigen die unterschiedlichen Ausbeuten bei der Hydrierung von 1,1'-(Oxybis(ethane-2,1-diyl)) bis (pyrrolidin-2-on in Gegenwart von unterschiedlichen Katalysatorsystemen bei unterschiedlichen weiteren Parametern

**Tabelle 3:**

| Versuch | Druck (bar) | Temperatur (°C) | Katalysator | Lösungsm ittel /Additiv | Rktzeit (h) | Umsatz(%) | Ausbeute (GC %) |
|---|---|---|---|---|---|---|---|
| 1 | 80 | 170 | Ra-Co | Monoglyme 90% | 12 | 100 | 51,3 |
| 2 | 260 | 170 | Ra-Co | - | 36 | 98,9 | 70,3 |
| 3 | 260 | 170 | Ra-Co | Monoglyme 90% | 24 | 100 | 71,5 |
| 4 | 180 | 170 | Ra-Co | - | 60 | 100 | 60,9 |
| 5 | 180 | 200 | Ra-Co | - | 15 | 87,6 | 26,5 |
| 6 | 180 | 200 | Ra-Co | Dibutylamin 10% | 15 | 95,3 | 28,0 |
| 7 | 180 | 200 | Cu-Cr/Ba E 108 P | - | 15 | 61,8 | 10,5 |
| 8 | 180 | 240 | Cu-Cr/Ba E 108 P | - | 15 | 77,2 | 19,3 |
| 9 | 180 | 240 | Cu-Cr/Ba E 108 P | Hexan 50% | 15 | 100 | 66,3 |
| 10 | 180 | 240 | Cu-Cr/Ba E 108 P | Hexan 25% | 12 | 100 | 59 |

**Tabelle 4:**

| Versuch | Druck (bar) | Temperatur (°C) | Katalysator | Lösungsm ittel / Additiv | Rktzeit (h) | Umsatz (%) | Ausbeute (GC %) |
|---|---|---|---|---|---|---|---|
| 13 | 180 | 200 | Cu-Cr/Mn (Cu 1950 P) | - | 15 | 12,0 | 0,3 |
| 14 | 180 | 200 | Cu-Cr (Cu 1885 P) | - | 15 | 16,9 | 0,7 |

**Tabelle 5:**

| Versuch | Druck (bar) | Temperatur (°C) | Katalysator | Lösungsmittel / Additiv | Belastung (kg/L*h) | Umsatz (%) | Ausbeute (GC %) |
|---|---|---|---|---|---|---|---|
| 15 | 200 | 170 | Ra-Co | - | 0,21 | 87,8 | 25,4 |
| 16 | 200 | 190 | Ra-Co | - | 0,21 | 99,0 | 51,8 |
| 17 | 200 | 190 | Ra-Co | THF (25%) | 0,21 | 99,3 | 55,2 |
| 18 | 200 | 190 | Ra-Co | THF (50%) | 0,14 | 99,9 | 48,1 |

## Patentansprüche

1. Verfahren zur Herstellung von Bispyrrolidin-Verbindungen der Formel I 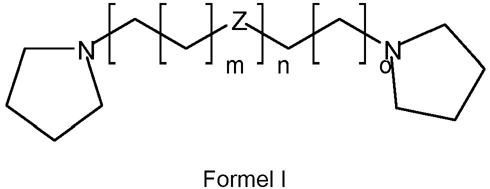 mit
Z ausgewählt aus der Gruppe von Sauerstoff und Kohlenstoff,
n eine ganze natürliche Zahl im Bereich von 0 bis 9 ist,
m und o unabhängig voneinander eine natürliche Zahl im Bereich von 1 bis 12 sind,
wobei in Schritt I) γ-Butyrolacton mit Diaminen der Formel II 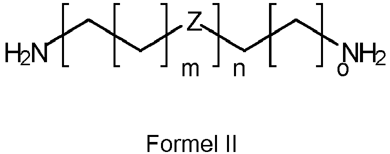 wobei Z, n, m und o die obige Bedeutung haben, zu Bisamiden der Formel III 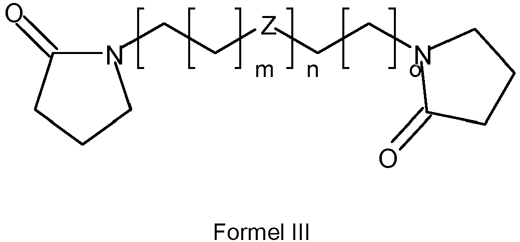 umgesetzt werden und anschließend
im Schritt II) die Bisamide der Formel III katalytisch zu den Bispyrrolidin-Verbindungen der Formel I hydriert werden.

2. Verfahren nach Anspruch 1, wobei die Umsetzung des γ-Butyrolactons mit der Verbindung II in Schritt I) bei Temperaturen im Bereich von 100 bis 300 °C durchgeführt wird und gleichzeitig oder anschließend das überschüssige Wasser destillativ entfernt wird.

3. Verfahren nach einem der Ansprüche 1 bis 2, wobei der Schritt II) in Gegenwart eines Hydrierkatalysators durchgeführt wird, bei dem der Hydrierkatalysator wenigstens ein Metall ausgewählt aus der Gruppe von Cu, Cr, Ni, Co, Fe, Pt, Pd, Re, Ru, und Rh enthält.

4. Verfahren nach einem der Ansprüche 1 bis 3, wobei in Schritt II) mit einem heterogenen Katalysator gearbeitet wird

5. Verfahren nach einem der Ansprüche 1 bis 4, wobei Schritt II) in Gegenwart eines Raney Cobalt, Raney Nickel oder dotierten oder undotierten Katalysatortyps von Cu-Cr durchgeführt wird.

6. Verfahren nach einem der Ansprüche 1 bis 5, wobei die Hydrierung in Schritt II) in Gegenwart eines Raney Cobalt Katalysators oder eines dotierten Katalysatortyps von Cu-Cr bei Temperaturen im Bereich von 100 bis 300°C und bei einem Wasserstoffdruck im Bereich von 100 bis 350 bar durchgeführt wird.

7. Verfahren nach einem der Ansprüche 1 bis 6, wobei in den Bispyrrolidin-Verbindungen der Formel I Z= Sauerstoff ist und n= 1 oder 2 ist und m=o=1 ist.

8. Verfahren nach einem der Ansprüche 1 bis 7, wobei die Verbindungen der Formel IB bei denen unabhängig voneinander -X1 und -X2 ausgewählt sind aus der Gruppe von H und Doppelbindung Sauerstoff, wobei aber wenigstens -X1 oder-X2 für Wasserstoff steht und die nach Schritt II neben den Bispyrrolidin-Verbindungen der Formel I erhalten werden können in einem weiteren Schritt III) noch einmal katalytisch hydriert werden.

9. Verfahren nach einem der Ansprüche 1 bis 8, wobei die Hydrierung von Schritt II) und III) mit dem gleichen Katalysator durchgeführt wird.

10. Verfahren nach einem der Ansprüche 1 bis 9, wobei die Hydrierung von Schritt II) und III) in einem Schritt erfolgen kann.

11. Verfahren nach einem der Ansprüche 1 bis 10, wobei die Verbindung nach Formel I der Bispyrrolidin-ether der Formel IA ist

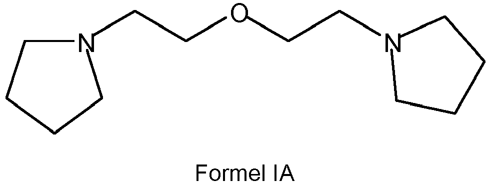

## Claims

1. A method for preparing bispyrrolidine compounds of the formula I 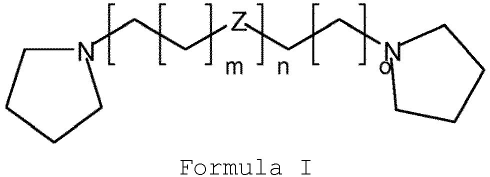 where
Z is selected from the group consisting of oxygen and carbon,
n is a whole natural number in the range from 0 to 9,
m and o are independently a natural number in the range from 1 to 12,
wherein, in step I), γ-butyrolactone is reacted with diamines of the formula II 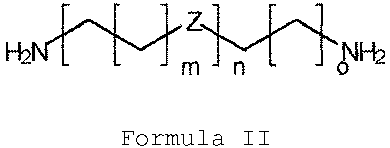 where Z, n, m and o are as defined above, to form bisamides of the formula III 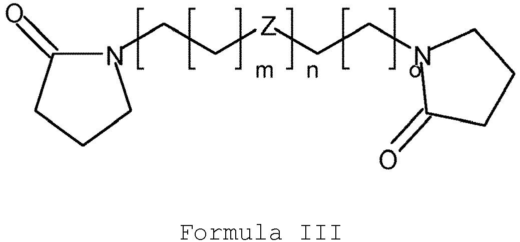 and then,
in step II), the bisamides of the formula III are catalytically hydrogenated to form the bispyrrolidine compounds of the formula I.

2. The method according to claim 1, wherein the reaction of γ-butyrolactone with compound II in step I) is carried out at temperatures in the range from 100 to 300°C with simultaneous or subsequent removal of the excess water by distillation.

3. The method according to either of claims 1 and 2, wherein step II) is carried out in the presence of a hydrogenation catalyst, in which the hydrogenation catalyst comprises at least one metal selected from the group consisting of Cu, Cr, Ni, Co, Fe, Pt, Pd, Re, Ru and Rh.

4. The method according to any of claims 1 to 3, wherein step II) is carried out using a heterogeneous catalyst.

5. The method according to any of claims 1 to 4, wherein step II) is carried out in the presence of a Raney cobalt, Raney nickel or doped or undoped catalyst of the Cu-Cr type.

6. The method according to any of claims 1 to 5, wherein the hydrogenation in step II) is carried out in the presence of a Raney cobalt catalyst or of a doped catalyst of the Cu-Cr type at temperatures in the range from 100 to 300°C and at a hydrogen pressure in the range from 100 to 350 bar.

7. The method according to any of claims 1 to 6, wherein, in the bispyrrolidine compounds of the formula I, Z = oxygen, n = 1 or 2 and m = o = 1.

8. The method according to any of claims 1 to 7, wherein the compounds of the formula IB, in which -X1 and -X2 are independently selected from the group consisting of hydrogen and double-bond oxygen, but where at least one of -X1 or -X2 is hydrogen, and which may be obtained in step II in addition to the bispyrrolidine compounds of the formula I, may undergo a further catalytic hydrogenation in a further step III).

9. The method according to any of claims 1 to 8, wherein the hydrogenation in steps II) and III) is carried out using the same catalyst.

10. The method according to any of claims 1 to 9, wherein the hydrogenation in steps II) and III) can be carried out in a single step.

11. The method according to any of claims 1 to 10, wherein the compound of the formula I is the bispyrrolidine ether of the formula IA

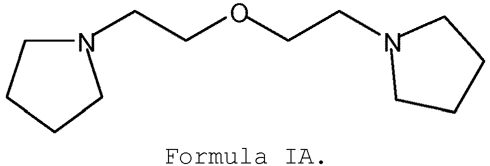

## Revendications

1. Procédé de préparation de composés bispyrrolidines de Formule I 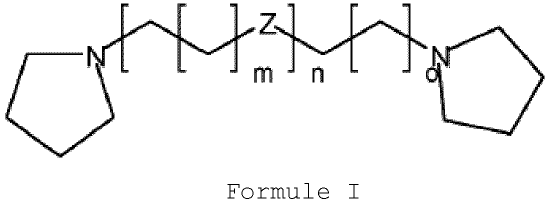 dans laquelle
Z est choisi dans le groupe consistant en l'oxygène et le carbone,
n représente un nombre entier dans la plage de 0 à 9,
m et o représentent indépendamment l'un de l'autre un nombre entier dans la plage de 1 à 12,
dans lequel, dans l'étape I), on fait réagir de la γ-butyrolactone avec des diamines de Formule II 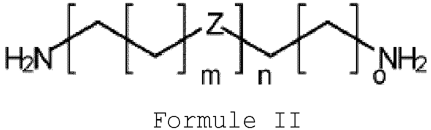 dans laquelle Z, n, m et o ont les significations données ci-dessus, pour obtenir des bisamides de Formule III 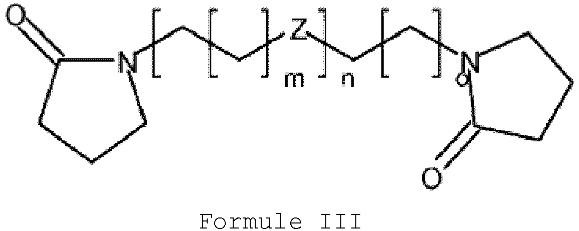 puis,
dans l'étape II), on hydrogène les bisamides de Formule III par voie catalytique pour obtenir les composés bispyrrolidines de Formule I.

2. Procédé selon la revendication 1, dans lequel la réaction de la γ-butyrolactone avec le composé II dans l'étape I) est mise en œuvre à des températures dans la plage de 100 à 300 °C, l'eau en excès étant, simultanément ou ultérieurement, éliminée par distillation.

3. Procédé selon l'une des revendications 1 à 2, dans lequel l'étape II) est mise en œuvre en présence d'un catalyseur d'hydrogénation, le catalyseur d'hydrogénation contenant au moins un métal choisi dans le groupe Cu, Cr, Ni, Co, Fe, Pt, Pd, Re, Ru et Rh.

4. Procédé selon l'une des revendications 1 à 3, dans lequel, dans l'étape II), on travaille avec un catalyseur hétérogène.

5. Procédé selon l'une des revendications 1 à 4, dans lequel l'étape II) est mise en œuvre en présence d'un cobalt Raney, d'un nickel Raney ou d'un type de catalyseur dopé ou non dopé de Cu-Cr.

6. Procédé selon l'une des revendications 1 à 5, dans lequel l'hydrogénation de l'étape II) est mise en œuvre en présence d'un catalyseur au cobalt Raney ou d'un type de catalyseur dopé de Cu-Cr à des températures dans la plage de 100 à 300 °C et sous une pression d'hydrogène dans la plage de 100 à 350 bar.

7. Procédé selon l'une des revendications 1 à 6, dans lequel, dans les composés bispyrrolidines de Formule I, Z = oxygène et n = 1 ou 2 et m = o = 1.

8. Procédé selon l'une des revendications 1 à 7, dans lequel les composés de Formule IB dans lesquels, indépendamment l'un de l'autre, -X1 et -X2 sont choisis dans le groupe consistant en H et une double liaison oxygène, mais au moins -X1 ou -X2 représentant un hydrogène, et qui, dans l'étape II, peuvent être obtenus en plus des composés bispyrrolidines de Formule I, sont encore une fois hydrogénés par voie catalytique dans une étape supplémentaire III).

9. Procédé selon l'une des revendications 1 à 8, dans lequel l'hydrogénation de l'étape II) et de l'étape III) est mise en œuvre avec le même catalyseur.

10. Procédé selon l'une des revendications 1 à 9, dans lequel l'hydrogénation de l'étape II) et de l'étape III) peut avoir lieu en une étape.

11. Procédé selon l'une des revendications 1 à 10, dans lequel le composé de Formule I est l'oxyde de bispyrrolidinyle de Formule IA
